(19) Europäisches Patentamt

European Patent Office

Office européen des brevets

(11) EP 0 717 642 B1

(12) EUROPÄISCHE PATENTSCHRIFT

(45) Veröffentlichungstag und Bekanntmachung des Hinweises auf die Patenterteilung:
15.04.1998 Patentblatt 1998/16

(21) Anmeldenummer: 94926867.6

(22) Anmeldetag: 23.08.1994

(51) Int. Cl.$^6$: A61M 25/00

(86) Internationale Anmeldenummer:
PCT/EP94/02787

(87) Internationale Veröffentlichungsnummer:
WO 95/07113 (16.03.1995 Gazette 1995/12)

(54) **KATHETER**

CATHETER

CATHETER

(84) Benannte Vertragsstaaten:
AT BE CH DE DK FR GB IT LI NL SE

(30) Priorität: 06.09.1993 DE 4330089

(43) Veröffentlichungstag der Anmeldung:
26.06.1996 Patentblatt 1996/26

(73) Patentinhaber:
GSF-Forschungszentrum für Umwelt und Gesundheit, GmbH
D-85758 Oberschleissheim (DE)

(72) Erfinder: FRIEDRICH, Peter
D-80496 München (DE)

(56) Entgegenhaltungen:
DE-A- 3 504 661          DE-U- 8 809 319
FR-A- 2 144 806          US-A- 5 112 301
US-A- 5 224 938

**Beschreibung**

Die Erfindung betrifft einen Katheter nach dem Oberbegriff des Patentanspruchs 1.

Verweilkatheter werden immer dann eingesetzt, wenn langfristig Infusionen notwendig werden. Dazu werden immer häufiger implantierbare Infusionspumpen eingesetzt. Die wesentlichen Vorteile sind: geringere infektiöse Komplikationen, größere Bewegungsfreiheit des Patienten usw.

Beim Einsatz implantierter Infusionspumpen stehen Katheterobstruktionen in der Häufigkeit der Komplikationen an erster Stelle. Zur Beseitigung des Katheterverschlusses muß die gesamte Pumpe explantiert werden, was einen großen chirurgischen Eingriff notwendig macht. Neben den Kosten eines solche Eingriffs resultiert daraus eine Verringerung der Akzeptanz der Pumpe beim Patienten.

Ursache für diese Katheterverschlüsse ist eine Thrombusenstehung im Katheter. Diese wird ausgeglöst durch einen Einstrom von Blut in das Katheterlumen.

Im allgemeinen sind an den Kathetern Infusionspumpen angeschlossen, die volumenkonstant arbeiten. Das ist erforderlich, um eine bestimmte Medikamentenmenge in einer bestimmten Zeit zu verabreichen. Ändern sich die Druckverhältnisse an der Kathersplitze, so wird der Druckausgleich im Katheter bei einer vorhandenen Compliance von einer Volumenänderung im Katheter begleitet.

Durch die Verwendung elastischer Materialien ist ein Volumenstrom in das Katheterlumen möglich. Liegt ein Katheter mit Frontöffnung gerade in Strömungsrichtung, so muß für die Förderung des Infusats ein Druck $P_k$ im Katheter erzeugt werden, der größer als der Außendruck ist:

$$P_k > P_{stat} - (P_{dyn} - P_{verl})$$

$P_k$ - Katheterinnendruck, $P_{stat}$ - statischer Umgebungsdruck, $P_{dyn}$ - dynamischer Umgebungsdruck, $P_{verl}$ - Druckverlust

Der statische Druck der Außenströmung muß überwunden werden. Da der Katheter im Prinzip ein Pitotrohr in umgekehrter Richtung darstellt, ist der dynamische Druck vom statischen Druck abzuziehen. Der dynamische Druck verringert sich um den Druckverlust, der sich aus der nicht idealen, d.h. reibungsfreien Umströmung des Katheters ergibt. Der notwendige Förderdruck im Katheter hat bei kleinen Infusionsraten eine sehr kleine dynamische Komponente, so daß der statische Druck bestimmend ist.

Da Volumenkonstanz für das Infusat gilt, wird bei steigenden Umgebungsdruck Umgebungsflüssigkeit in das Katheterlumen gedrückt, um so mehr, je größer die Compliance des System ist. Bis die Infusionspumpe wieder einen inneen Überdruck hergestellt hat, können sich Blutbestandteile an der Katheterinnenwand, die oft aus einem anderen, weniger biokompatiblen Material als die Außenwand gefertigt ist, anlagern.

Das implantierte Infusionssystem ist im Körper des Patienten verschiedenen Druckschwankungen ausgesetzt. Durch die Compliance des Infusionssystems (Pumpe mit Katheter) wird bei äußeren Überdruck Blut in den Katheter gedrückt. Im Katheterlumen entsteht durch die lange Verweilzeit ein Thrombus der den Katheter verschließt. Ein einfacher Schlitz im Katheter, wie beim Groshong-Katheter, verhindert das Eindringen von Blut bei äußeren Überdruck nicht.

Eine weitere Ursache für eine Thrombosebildung am Katheter ist eine ungünstige Form des Katheterendes. Die Thromben am Katheterende können außerdem ablösen und zu einen thrombotischen Verschluß eines Gefäßes führen, was ernsthafte gesundheitliche Schäden beim Patienten verursachen kann.

Ein Katheter ist aus der DE 32 01 954 A1 bekannt. Das dort beschriebene Ventil wird durch einen geraden Schnitt mit den o. a. Nachteilen gebildet.

Aus der DE 35 04 661 A1 ist ein Katheter für die Dauerapplikation bekannt welches an seiner Spitze ein Entenschnabelventil aufweist.

Des weiteren ist aus der DE 35 40 949 ein Katheter bekannt, welcher am patientenseitigen Ende einen starren Einsatzkörper trägt, welcher zusammen mit dem Katheterschlauch ein Ventil bildet.

Aus der US-A-5,224,938 ist ein Katheter der gattungsgemäßen Art bekannt, bei dem jedoch größere Toträume im Innern vorhanden sind und an dessen Spitze es leicht zu Trombosebildung kommen kann.

Aufgabe der Erfindung ist es, einen Katheter der e.A. zur Verfügung zu stellen, bei dem die Bildung von Thromben minimiert ist.

Gelöst wird diese Aufgabe durch die Merkmale des Patentanspruchs 1. Die Unteransprüche beschreiben vorteilhafte Ausgestaltungen des Verfahrens.

Die Erfindung wird im folgenden an Hand eines Ausführungsbeispiels mit Hilfe der Figuren näher erläutert.

Dabei zeigt Fig. 1 schematisch Aufsicht und Schnitt eines Katheterendes. Fig. 2 zeigt ein Ventil geöffnet und geschlossen, während in Fig. 3 zwei verschieden ausgeformte Katheterspitzen dargestellt sind.

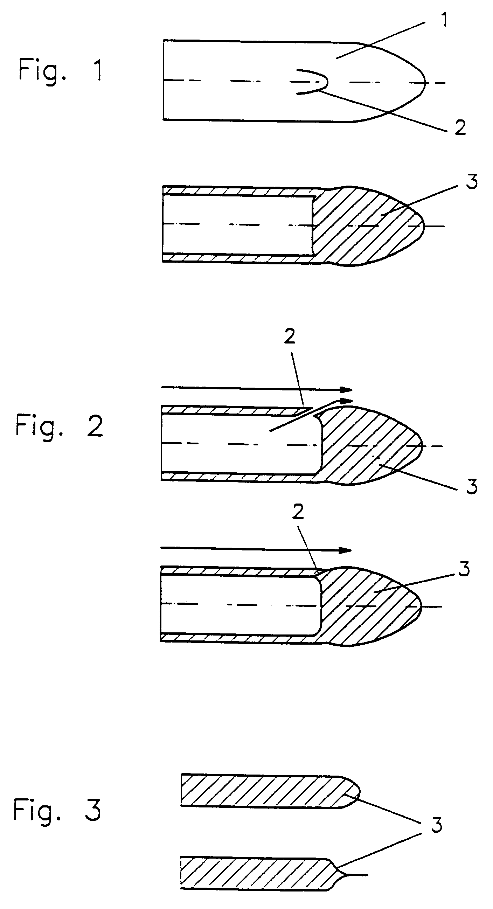

Die Fig. 1 zeigt oben die Aufsicht des distalen Endes eines Katheters 1 mit einem parabelförmigen Ventil 2. Der Katheter besteht hier aus einem Silikonschlauch, dessen Ende geschlossen und kegelförmig ausgestaltet ist.

Der Schnitt, welcher das Ventil bildet, ist so ausgeführt, daß zwischen der Flächennormalen und der Schnittlinie nach außen ein Winkel von ca. 30° liegt. Der Winkelbereich kann 15 bis 80° betragen. Durch den Schnitt bildet der Katheterschlauch eine rampenförmige Auflage für den parabelförmigen Ventillappen, die verhindert, daß der Ventillappen bei äußeren Überdruck ins innere des Katheters gedrückt wird. Die Schnittflächen bilden die Dichtfläche des Ventils.

Der untere Teil der Figur zeigt einen schnitt durch das Katheterende.

Die Figur 2 zeigt zwei Längsschnitte durch das Katheterende im Bereich des Scheitels des Ventils 2. Der obere Schnitt zeigt das Ventil im geöffneten Zustand (der 2. Strömungspfeil zeigt den durch Überdruck von innen zudosierten Flüssigkeitsstrom). Der untere Schnitt zeigt das Ventil im geschlossenen Zustand (durch äußeren Überdruck). Die Verengung des Katheterlumens zur Vermeidung eines Totraumes am Ventil 2 ist hier nicht dargestellt. Bei dieser Figur ist am Katheterende im Bereich des Ventils 2 eine Einschnürung des Katheters angedeutet. Diese Einschnürung stabilisiert das Ventil im geschlossenen Zustand.

Es ist sinnvoll am distalen Katheterende noch ein oder mehrere Ventile vorzusehen. Diese Ventile sollten dann so geschnitten sein, daß sie erst bei größerem Druck öffnen, als das erste Ventil. Sie können dann, wenn das erste Ventil durch Thromben blockiert ist, dessen Funktion übernehmen. Erreicht wird das spätere Öffnen (bei höherem Innendruck) durch eine andere Schnittführung (z. B. kürzer).

Die Figur 3 zeigt oben ein Katherende mit einem paraboloidähnlichen stumpfen Kegel für Anwendungen bei geringer Thromboseneigung und unten ein Katheterende mit spitzem Kegel, bei welchem auf der Kuppe eines stumpfen Kegels eine ausgezogene Spitze sitzt, für Anwendungen bei hoher Thromboseneigung.

Die Geometrie einer Katheterspitze, welche eine Thrombusbildung minimiert, ergibt sich aus den am Katheterort herrschenden Blutströmungsverhältnissen. Diese werden berechnet, indem die Geometrie eines in einem Blutgefäß liegenden Katheters ohne Spitze (einfache Schlauchöffnung) diskretisiert wird, d.h. in genügend kleine Elemente aufgeteilt wird (finite Elemente). Nach Festlegung der Randbedingungen, wie Einstromgeschwindigkeit, Infusionsgeschwindigkeit, Viskosität usw. wird ein nichtlineares Gleichungssystem aufgestellt (Navier-Stokes Gleichungen). Die Anwendung der Finiten Elemente Methode ergibt ein System von Gleichungen:

$$L(U)*U=F$$

L ist die globale Systemmatrix, U der globale Geschwindigkeitsvektor und F enthält die Randbedingungen des Strömungsgebiets und die Stoffeigenschaften von Blut. Um die Veränderung der Viskosität erfassen zu können, wurde in jedes Element ein Quellterm installiert. Das Feld für diese Quelle am Strömungseintritt ist mit Null initialisiert. Die Lösung des Gleichungssystems hat zur Folge, daß sich der Wert des Quellterms in Abhängigkeit von der Konvektion und Diffusion im berechneten Strömungsgebiet verändert. Aus diesem Wert wird nach jedem Lösungsschritt des iterativen Lösungsvorganges die Viskosität berechnet, die wiederum die Strömung beeinflußt. Die Gleichung mit der Quellterm und Viskosität verknüpft werden, entspricht der Reaktionsgleichung der Fibrinpolymerisation und kann entsprechend der Reizung des Koagulationssystems in vivo (beim Patienten) eingestellt werden.

Nach der Lösung des gesamten Gleichungssystems wird die Viskositätsverteilung graphisch dargestellt. Die Form des Gebietes der maximalen Viskosität am Katheter entspricht der Form der zu verwendenden Katheterspitze.

Um eine Anpassung der Spitze bei geringfügigen Lageveränderungen im Blutgefäß zu erreichen, muß die Spitze aus einem genügend weichen Material (Silikon) gefertigt werden. Die entstehenden Druckunterschiede führen zu einer Anpassung der Ausrichtung der Katheterspitze in der Art einer Wetterfahne. Durch das weichere Material werden auch Verletzungen bei Bewegungen des Katheters vermieden.

## Patentansprüche

1. Katheter (1) aus elastischem Material, welcher an seinem distalen Ende rotationssymmetrisch ausgebildet ist, und welcher eine seitliche Öffnung aufweist, die mit einem Ventil (2) versehen ist, welches sich bei äußeren Überdruck schließt, wobei das Ventil durch einen parabelförmigen Schnitt in der Katheterwand, dessen Scheitel zum distalen Katheterende weist und dessen Schnittlinie schräg zur Katheterwand verläuft, gebildet wird, dadurch gekennzeichnet, daß sich das Lumen des Katheters (1) zum Ventil (2) hin derart verengt, daß am Ventil (2) kein Totraum entsteht.

2. Katheter nach Anspruch 1, dadurch gekennzeichnet, daß mindestens ein weiteres Ventil im Bereich des distalen Katheterendes vorhanden ist.

3. Katheter nach Anspruch 1 oder 2, dadurch gekennzeichnet, daß das distale Ende (3) als stumpfer Kegel ausgebildet ist, wobei die Form der Katheterspitze der Form des Gebietes maximaler Viskosität am Katheter entspricht.

4. Katheter nach Anspruch 1 oder 2, dadurch gekennzeichnet, daß das distale Ende (3) als spitzer Kegel ausgebildet ist, wobei die Form der Katheterspitze der Form des Gebietes maximaler Viskosität am Katheter entspricht.

5. Verwendung des Katheters nach einem der Ansprüche 1 bis 4 als Verweilkatheter.

## Claims

1. Catheter (1) formed from resilient material, which has a rotationally symmetrical configuration at its distal end and has a lateral aperture provided with a valve (2), which closes in the event of excess external pressure, the valve being formed by a parabolic incision in the catheter wall, the crown of which incision points towards the distal end of the catheter, and the secant of which incision extends inclinedly relative to the catheter wall, characterised in that the internal diameter of the catheter (1) reduces towards the valve (2) in such a manner that space is produced at the valve (2).

2. Catheter according to claim 1, characterised in that at least one additional valve is provided in the region of the distal end of the catheter.

3. Catheter according to claim 1 or 2, characterised in that the distal end (3) is configured as a truncated cone, the configuration of the catheter tip corresponding to the configuration of the area of maximum viscosity on the catheter.

4. Catheter according to claim 1 or 2, characterised in that the distal end (3) is configured as a pointed cone, the configuration of the catheter tip corresponding to the configuration of the area of maximum viscosity on the catheter.

5. Use of the catheter according to one of claims 1 to 4 as a time-delay catheter.

**Revendications**

1. Cathéter (1) en matériau élastique, qui est formé à son extrémité distale par symétrie de rotation, et qui présente une ouverture latérale munie d'une soupape (2) qui se ferme en cas de surpression extérieure, la soupape étant formée par une fente en forme de parabole dans la paroi du cathéter, à son sommet, par rapport à l'extrémité distale du cathéter, et dont la ligne de découpe court obliquement à la paroi du cathéter,
caractérisé en ce que
la lumière du cathéter (1) se rétrécit par rapport à la soupape (2) de manière qu'il n'apparaisse pas d'espace mort sur la soupape (2).

2. Cathéter selon la revendication 1,
caractérisé en ce qu'
au moins une autre soupape est présente dans la zone de l'extrémité distale du cathéter.

3. Cathéter selon la revendication 1 ou 2,
caractérisé en ce que
l'extrémité distale (3) est formée en sommet tronqué, la forme de la pointe du cathéter correspondant à la forme de la zone de viscosité maximale sur le cathéter.

4. Cathéter selon la revendication 1 ou 2,
caractérisé en ce que
l'extrémité distale (3) est formée en cône pointu, la forme de la pointe du cathéter correspondant à la forme de la zone de viscosité maximale sur le cathéter.

5. Utilisation du cathéter selon l'une des revendications 1 à 4 comme cathéter à demeure.

Fig. 1

Fig. 2

Fig. 3